# EUROPEAN PATENT APPLICATION

(11) **EP 2 738 704 A1**
(43) Date of publication of application: **04.06.2014**
(21) Application number: 12306505.4
(22) Date of filing: 03.12.2012
(51) Int. Cl.: G06F 19/12, G06F 19/26

(54) **A computer-implemented method for simulating, in a three-dimensional scene, the evolution of biological data**

(71) Applicant: Dassault Systèmes, 78140 Velizy Villacoublay (FR)
(72) Inventor: Pradalier, Sylvain, 91160 CHAMPLAN (FR); Kervizic, Gwenael, 92190 MEUDON (FR)
(74) Representative: Brunelli, Gérald

(57) **Abstract**

A computer-implemented method for simulating, in a three-dimensional scene, the evolution of biological data comprising the steps of :
- displaying an initial biological interaction network (BIN) of biological entities ; and
- determining biological data for said interaction network (BIN) ;

characterized in that the method further comprises the step of :
- displaying, in a three-dimensional scene, a biological data of each entity in a first layout (2, 3, 4, 5), a first layout comprising several levels, a level corresponding to the value of the biological data at a time instant (t).

## Description

The invention relates to the field of computers programs and systems, and more specifically to the field of simulating of the evolution of biological data, that can be generated by simulating a biological interactions network, or can be derived from experiments.

A biological interactions network or BIN describes some biological phenomenon and can be visualized as a graph, whose nodes represent the biological entities involved in the phenomenon to represent and whose edges represent the biological interactions between the entities.

Biological data can be originated either from experiments performed by biologists or pharmacologists, or from computer simulation.

Two main sorts of data can be identified : time series and global observables, which sometimes can be combined.

Times series correspond typically to computer simulation traces or series of experimental measures along time (DNA, chips, ...), and global observables can either be experimental measures (i.e. triggering of the apoptosis, measure of tumor diameter, ...) or deduced using mathematical expressions, such as sums or means, upon values of pathway elements.

The most commonly used approach by both academicals and industrial products to present these data is to use plots, or curves in small windows. To illustrate such a realization, it is possible to cite the software Cell-Designer (trademark) or the software SimBiology (trademark).

Some other products also provide a graphical representation of the data at one particular instant, using colorations of the biological interactions network entities and interactions. The different colors represent different data values, like illustrated by the software CellNetAnalyser (trademark).

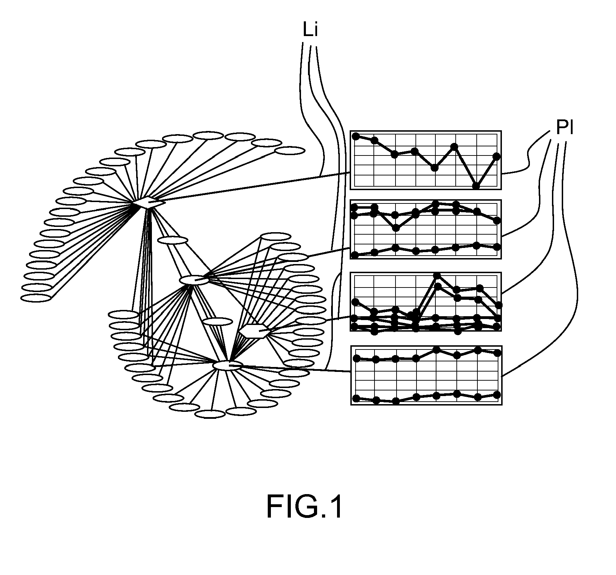

It also exists software wherein plots PI were presented over the biological interactions network and graphically linked Li to the corresponding entity, like PathWayStudio of Ariadne Genomics, like illustrated on figure 1.

The main problem with the plot approach is that the biological interactions network authoring and visualization, and the visualization and comparison of experimental and simulation data are two separated experiences. Very often the biological interactions network authoring and the data visualization occur in separate windows or frames, or the data and the biological interactions network cannot be visualized at the same time.

So efforts are required from the user in order to perform the correspondence between the entities of the biological interactions network and the data in the plots.

To overcome this difficulty, the use of colorations was proposed. However, the main problem with the coloration approach is the loss of the time perspective. The data can be visualized only at some given instant.

In this mode, the data evolution (typically increase or decrease) cannot be visualized in one view, rather it requires a succession of views, typically an animation. Even more, comparisons can be performed only at one time instant.

The approach of the software Caleydo (trademark), which allows to visualize a two-and-half dimensional scene and PathwayStudio solve partially these issues, in the sense that the time perspective is preserved, and there is a graphical link between a plot and the data it corresponds to.

However, data visualization and biological interaction network authoring and visualization are still two different experiences: plots are displayed in panels over the biological interaction network.

Moreover the link between a data and its corresponding entity is not an immediate information: links need to be followed, which can become quite awkward in the case of large networks.

Thus, software in the field of biological interaction network do not offer functionalities to easily compared experimental data or computed data with others experimental data or computed data in the context of biological interaction networks.

A goal of the invention is to provide a computer-implemented method and a system to overcome the above mentioned problems.

It is proposed, according to one aspect of the invention, a computer-implemented method for simulating, estimating, analyzing and/or displaying, in a three-dimensional scene, the evolution of biological data comprising the steps of :
- displaying an initial biological interaction network of biological entities ;
- determining biological data for said interaction network ; and
- displaying, in a three-dimensional scene, a biological data of each entity in a first layout, a first layout comprising several levels, a level corresponding to the value of the biological data at a time instant.

Thus, such a method allows to visualize the correspondence between data and biological interaction network entities in an immediate way. There is neither graphical links to be followed neither any effort required from the user to understand the correspondence between data and biological interaction network entities.

This is possible by the use a layout in a three-dimensional scene, which provides a new dimension to display a specific type of information to the user (like simulation data).

This method allows to avoid the use of graphical links (like the links used in Caleydo), and allows to keep track of the biological network structure while visualizing the data. Visualization through graphical shapes is intuitive for the user, and allows to easily navigate through the different behavior, or, in other words, at different period, in the biological interaction network.

For example, the layout is displayed as a column.

Such a way, the user can easily analyze/study the time evolution of said biological interaction network entities through the evolution of their graphical representations.

For example, the levels of a layout can be displayed in a continuous manner.

Alternatively the levels of a layout can be displayed in a discretized manner.

In an embodiment, the method further comprising, the steps of :
- creating a virtual node of the biological interaction network corresponding to an information representative of a biological data of several biological entities of the initial biological interaction network ; and
- displaying, in the three-dimensional scene, a layout comprising several levels, a level corresponding to the value of said information at a time instant.

Thus, it is possible for the user to access an information representative of a biological data of several biological entities of the initial biological interaction network.

Furthermore, said information can be a function of a biological data of the several biological entities of the initial biological interaction network.

Thus, it is possible to represent a function, like a sum or a mean of biological entity or entities of the initial biological interaction network.

For example, said information can be representative of the toxicity of the biological entities of the initial biological interaction network

In an embodiment, the method further comprising, the step of displaying an adjusting layout comprising means to scroll the displayed time range of the all layouts and/or comprising means to highlight a same time instant of each layout.

The user can then choose a period to study and an instant to highlight.

In an embodiment, the method further comprises a step of displaying, in the three-dimensional scene, a biological data of each entity in a second layout, a second layout comprising several levels, a level corresponding to the value of the biological data at a time instant, one of the first layout and the second layout represents simulation values and the other one represents experiment values.

Thus it is easy and instantaneous to compare this additional information to previous displayed information (like interactions between biological interaction network entities or experimental data).

The method can further comprise a step of switching the display of a type of layouts, among first and second layouts, between a two-dimensional aspect and a three-dimensional aspect.

The switching can be effected by, for example by double-clicking on a type of layouts.

For example, a type of layout can have a cylindrical aspect in a three-dimensional displaying, and a concentric rings aspect in a two-dimensional displaying.

Thus the difference between the two types of layouts is immediate.

The number of displayed levels can be configurable.

Thus, it is easy and instantaneous to change the number of displayed levels or the first layouts.

It is proposed, according to another aspect of the invention, a computer-readable medium having computer-executable instructions to cause the computer system to perform the method for simulating, in a three-dimensional scene, the evolution of biological data as described above.

It is proposed, according to another aspect of the invention, a computer program product, stored on a computer readable medium, for simulating the evolution of biological data in a three-dimensional scene of a computer-aided system, comprising code means for causing the system to take the steps as described above.

It is proposed, according to another aspect of the invention, an apparatus for simulating the evolution of biological data in a three-dimensional scene of a computer-aided system comprising means for implementing the steps of the method as described above.

The invention will be better understood with the study of some embodiments described by way of non-limiting examples and illustrated by the accompanying drawings wherein :
- figure 1 illustrates an example of software of biological interactions network ; and
- figures 2 to 6 illustrate the functioning of the invention, according to different aspects of the invention.

Following figures explain more in details the functioning of the present invention.

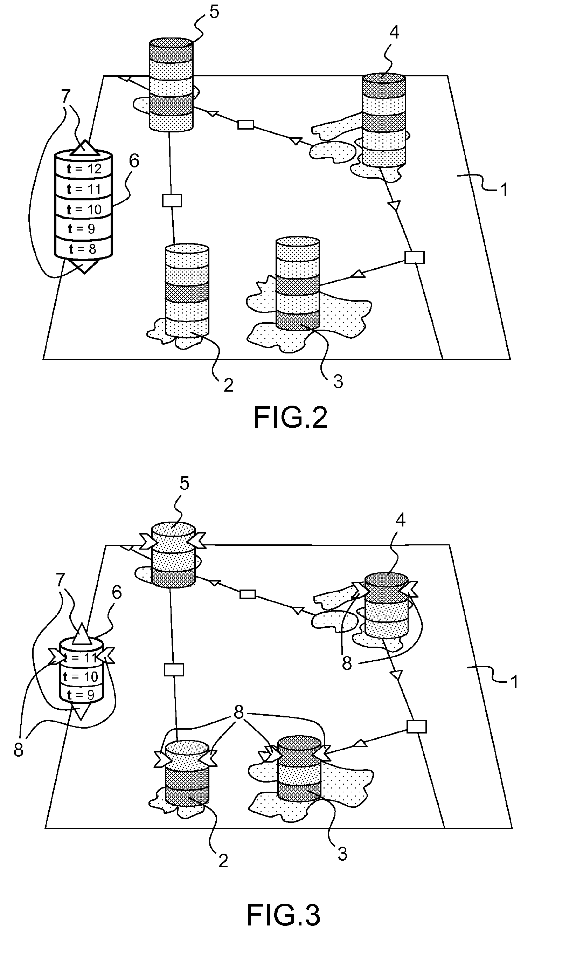

On figure 2, is represented, in a three dimensional scene, a biological interaction network or BIN of biological entities, according to an aspect of the invention.

The white plane 1 contains a subpart of the biological interaction network (in grey). Four layouts, for example four cylindrical columns 2, 3, 4 and 5 displaying biological data corresponding to each of four corresponding entities. Five levels are represented, corresponding at five successive time instants.

Here are represented five discrete levels, but, alternatively, the levels can be displayed in a continuous manner.

On the left side is also represented an adjusting layout 6 comprising means to scroll the displayed time range of the all layouts 2, 3, 4 and 5, for example vertical arrows 7.

On figure 3, the adjusting layout 6 furthermore comprises means to highlight a same time instant of each layout 2, 3, 4 and 5, for example horizontal arrows 8. Like illustrated a time instant is highlighted, on the example of figure 3, the time instant highlighted is t=11. Indeed, same horizontal arrows 8 are disposed in front of the highlighted instant for all the layouts like on the adjusting layout 6.

On figures 3 to 6, three successive levels are represented, to display values of data at t=9, t=10 and t=11.

By pressing the up and down arrows 7 of the slider the user can access to the data at other period, and with horizontal arrows 8 the user can highlight a particular time instant.

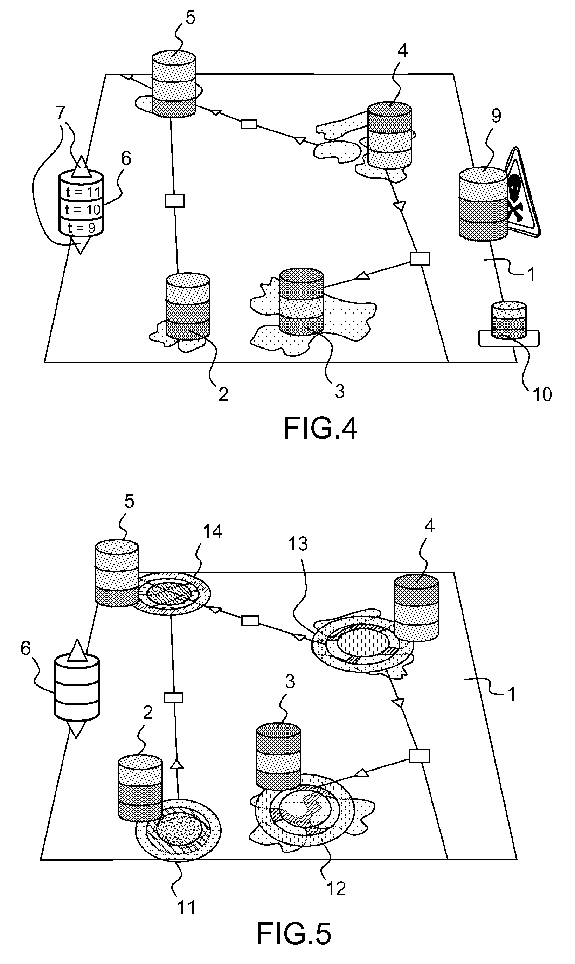

On figure 4, is also represented two additional virtual nodes to visualize global information, for instance, the two additional cylinders 9 and 10 can be used respectively to represent the variation of a toxicity data and of the value of a mathematical formula corresponding to an observable of the underlying biological system.

Like illustrated on figure 5, is displayed, in the three-dimensional scene, a biological data of each entity in a second layout 11, 12, 13, 14, a second layout comprising several levels, a level corresponding to the value of the biological data at a time instant, one of the first layout and the second layout represents simulation values and the other one represents experiment values.

On the example, the second layouts 11, 12, 13, 14 are represented in a two-dimensional aspect, with concentric rings.

On each layout, it is possible, for example by double-clicking on the layout, to switch between a two-dimensional aspect and a three-dimensional aspect of a type of layout (first layouts together or second layouts together).

Furthermore, the number of displayed levels can be configurable, and thus, it is easy and instantaneous to change the number of displayed levels or the first layouts.

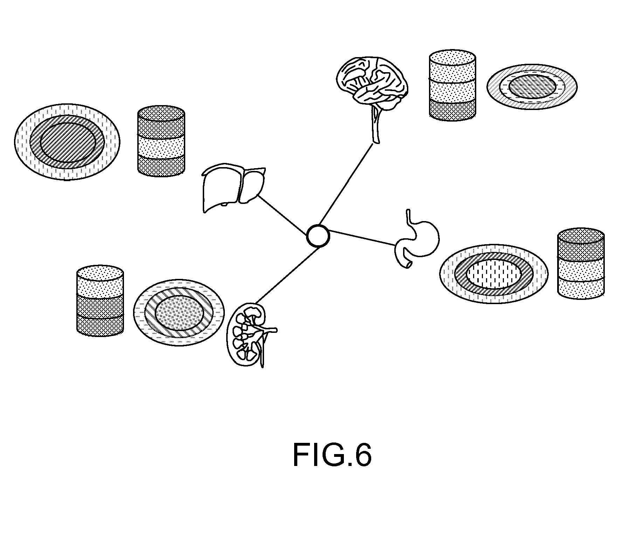

The present invention can be used in the fields of pharmacokinetics and pharmacodynamics like illustrated on figure 6.

Thus the present invention provides a computer-implemented method and a system for easily compared experimental data or computed data with others experimental data or computed data in the context of biological interaction networks.

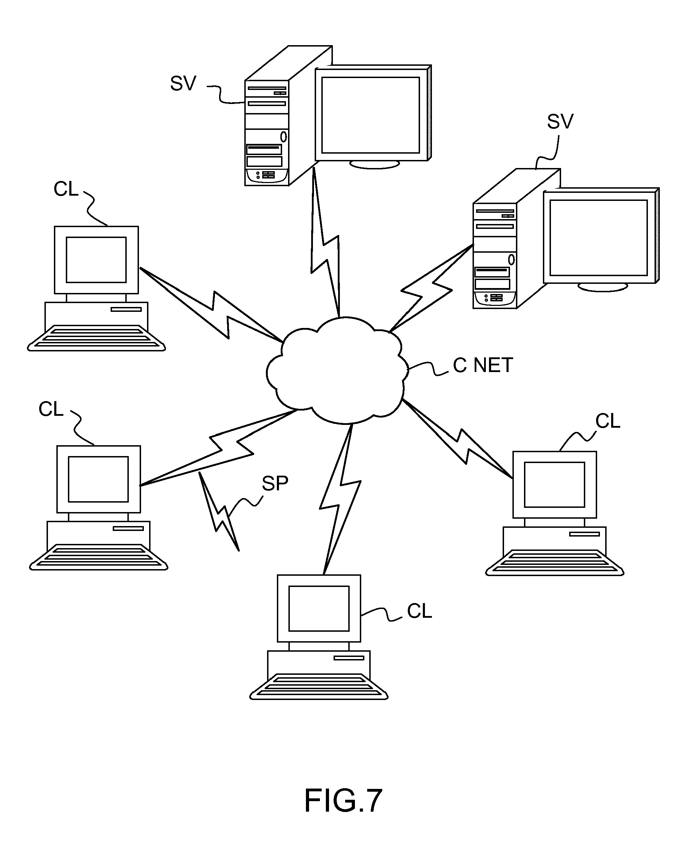

Figure 7 illustrates a computer network or similar digital processing environment in which the present invention may be implemented.

Client computer(s)/devices CL and server computer(s) SV provide processing, storage, and input/output devices executing application programs and the like. Client computer(s)/devices CL can also be linked through communications network CNET to other computing devices, including other client devices/processes CL and server computer(s) SV. Communications network 70 can be part of a remote access network, a global network (e.g., the Internet), a worldwide collection of computers, Local area or Wide area networks, and gateways that currently use respective protocols (TCP/IP, Bluetooth, etc.) to communicate with one another. Other electronic device/computer network architectures are suitable.

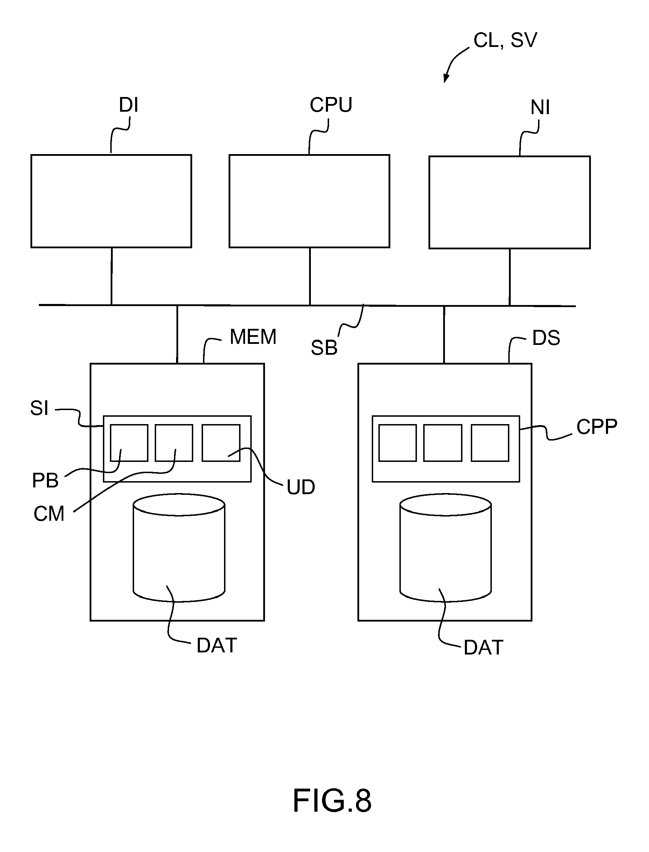

Fig. 8 is a diagram of the internal structure of a computer (e.g., client processor/device CL or server computers SV) in the computer system of Fig. 7. Each computer CL, SV contains system bus SB, where a bus is a set of hardware lines used for data transfer among the components of a computer or processing system. Bus SB is essentially a shared conduit that connects different elements of a computer system (e.g., processor, disk storage, memory, input/output ports, network ports, etc...) that enables the transfer of information between the elements.

Attached to system bus SB is I/O device interface DI for connecting various input and output devices (e.g., keyboard, mouse, displays, printers, speakers, etc.) to the computer CL, SV. Network interface NI allows the computer to connect to various other devices attached to a network (e.g., network CNET of Fig. 7).

Memory MEM provides volatile storage for computer software instructions SI and data CPP used to implement an embodiment of the present invention (e.g., a first path builder PB, means CM for computing a second path, an updater UD implementing the method discussed in Figs 1 to 6, and supporting code detailed above).

Disk storage DS provides non-volatile storage for computer software instructions SI and data DAT used to implement an embodiment of the present invention. Central processor unit CPU is also attached to system bus SB and provides for the execution of computer instructions.

In one embodiment, the processor routines SI and data DAT are a computer program product (generally referenced CPP), including a computer readable medium (e.g., a removable storage medium such as one or more DVD-ROM's, CD-ROM's, diskettes, tapes, etc...) that provides at least a portion of the software instructions for the invention system. Computer program product CPP can be installed by any suitable software installation procedure, as is well known in the art.

In another embodiment, at least a portion of the software instructions may also be downloaded over a cable, communication and/or wireless connection. In other embodiments, the invention programs are a computer program propagated signal product SP embodied on a propagated signal on a propagation medium (e.g., a radio wave, an infrared wave, a laser wave, a sound wave, or an electrical wave propagated over a global network such as the Internet, or other network(s)). Such carrier medium or signals provide at least a portion of the software instructions for the present invention routines/program CPP.

In alternate embodiments, the propagated signal is an analog carrier wave or digital signal carried on the propagated medium. For example, the propagated signal may be a digitized signal propagated over a global network (e.g., the Internet), a telecommunications network, or other network.

In one embodiment, the propagated signal is a signal that is transmitted over the propagation medium over a period of time, such as the instructions for a software application sent in packets over a network over a period of milliseconds, seconds, minutes, or longer.

In another embodiment, the computer readable medium of computer program product CPP is a propagation medium that the computer system CL may receive and read, such as by receiving the propagation medium and identifying a propagated signal embodied in the propagation medium, as described above for computer program propagated signal product.

Generally speaking, the term "carrier medium" or transient carrier encompasses the foregoing transient signals, propagated signals, propagated medium, storage medium and the like.

While this invention has been particularly shown and described with references to example embodiments thereof, it will be understood by those skilled in the art that various changes in form and details may be made therein without departing from the scope of the invention encompassed by the appended claims.

## Claims

1. A computer-implemented method for simulating, in a three-dimensional scene, the evolution of biological data comprising the steps of :
- displaying an initial biological interaction network (BIN) of biological entities ; and
- determining biological data for said interaction network (BIN) ; **characterized in that** the method further comprises the step of :
- displaying, in a three-dimensional scene, a biological data of each entity in a first layout (2, 3, 4, 5), a first layout comprising several levels, a level corresponding to the value of the biological data at a time instant (t).

2. Method according to claim 1, wherein the layout is displayed as a column (2, 3, 4, 5).

3. Method according to claim 1 or 2, wherein the levels of a layout are displayed in a continuous manner.

4. Method according to claim 1 or 2, wherein the levels of a layout (2, 3, 4, 5) are displayed in a discretized manner.

5. Method according to one of preceding claims, further comprising, the steps of :
- creating a virtual node of the biological interaction network (BIN) corresponding to an information representative of a biological data of several biological entities of the initial biological interaction network (BIN) ; and
- displaying, in the three-dimensional scene, a layout (9, 10) comprising several levels, a level corresponding to the value of said information at a time instant.

6. Method according to claim 5, wherein said information is a function of a biological data of the several biological entities of the initial biological interaction network (BIN).

7. Method according to claim 6, wherein said function comprises a sum and/or a mean of biological entity or entities of the initial biological interaction network (BIN).

8. Method according to claim 6 or 7, wherein said information is representative of the toxicity of the biological entities of the initial biological interaction network (BIN).

9. Method according to one of preceding claims, further comprising, the step of displaying an adjusting layout (6) comprising means to scroll (7) the displayed time range of the all layouts (2, 3, 4, 5) and/or comprising means to highlight (8) a same time instant of each layout.

10. Method according to one of preceding claims, further comprising a step of displaying, in the three-dimensional scene, a biological data of each entity in a second layout (11, 12, 13, 14), a second layout (11, 12, 13, 14) comprising several levels, a level corresponding to the value of the biological data at a time instant, one of the first layout (2, 3, 4, 5) and the second layout (11, 12, 13, 14) represents simulation values and the other one represents experiment values.

11. Method according to claim 11, further comprising a step of switching the display of a type of layouts, among first and second layouts, between a two-dimensional aspect and a three-dimensional aspect.

12. Method according to claim 11, wherein a type of layout has a cylindrical aspect in a three-dimensional displaying, and a concentric rings aspect in a two-dimensional displaying.

13. Method according to one of preceding claims, wherein the number of displayed levels is configurable.

14. A computer-readable medium having computer-executable instructions to cause the computer system to perform the method for simulating, in a three-dimensional scene, the evolution of biological data of anyone of claims 1 to 13.

15. A computer program product, stored on a computer readable medium, for simulating the evolution of biological data in a three-dimensional scene of a computer-aided system, comprising code means for causing the system to take the steps of anyone of claims 1 to 13.

16. An apparatus for simulating the evolution of biological data in a three-dimensional scene of a computer-aided system comprising means for implementing the steps of the method of anyone of claims 1 to 13.
